(19) Europäisches Patentamt

European Patent Office

Office européen des brevets

(11) Veröffentlichungsnummer: **0 683 392 A2**

# EUROPÄISCHE PATENTANMELDUNG

(21) Anmeldenummer: **95107439.2**

(22) Anmeldetag: **17.05.95**

(51) Int. Cl.⁶: **G01N 27/64**

(30) Priorität: **18.05.94 DE 4417366**

(43) Veröffentlichungstag der Anmeldung:
**22.11.95 Patentblatt 95/47**

(84) Benannte Vertragsstaaten:
**CH DE FR GB IT LI NL PT SE**

(71) Anmelder: **Druckfarbenfabrik Gebr. Schmidt GmbH**
**Gaugrafenstrasse 4-8**
**D-60489 Frankfurt (DE)**
Anmelder: **BRUKER-SAXONIA ANALYTIK GMBH**
**Permoserstrasse 15**
**D-04318 Leipzig (DE)**

(72) Erfinder: **Seng, Hans-Peter, Dr.**
**Memlingstrasse 5**
**D-12203 Berlin (DE)**
Erfinder: **Mehnert, Reiner, Prof. Dr.**
**Mittelstrasse 8**
**D-04416 Markkleeberg (DE)**
Erfinder: **Döring, Hans-Rüdiger, Dipl.-Phys.**
**Ludwigstrasse 131**
**D-04315 Leipzig (DE)**

(74) Vertreter: **Pfeifer, Hans-Peter, Dr.rer.nat.**
**Patentanwalt**
**Nowackanlage 15**
**D-76137 Karlsruhe (DE)**

(54) **Verfahren zum qualitätskontrollierten Veredeln einer Oberfläche mit einer strahlungsgehärteten Oberflächenveredelung.**

(57) Verfahren zum qualitätskontrollierten Veredeln von Oberflächen mit einer strahlungsgehärteten Oberflächenveredelung, bei welchem eine Veredelungsformulierung auf die Oberfläche aufgetragen und unter Einwirkung von Strahlung polymer vernetzt wird.

Um die Qualitätskontrolle nicht-destruktiv und mit verringertem Aufwand zu ermöglichen, wird vorgeschlagen, daß die Veredelungsformulierung eine mit der Ionenbeweglichkeitsspektrometrie nachweisbare protonenaffine Indikatorsubstanz enthält und die aus der bedruckten oder beschichteten Oberfläche nach der Strahlenhärtung entweichende Indikatorsubstanz mittels der Ionenbeweglichkeitsspektrometrie nachgewiesen wird.

EP 0 683 392 A2

Die Erfindung betrifft ein Verfahren zum qualitätskontrollierten Veredeln von Oberflächen mit einer polymeren Oberflächenveredelung, bei welchem eine strahlungshärtende Veredelungsformulierung auf die Oberfläche aufgetragen und unter Einwirkung von Strahlung polymer vernetzt und dadurch gehärtet wird.

Die Veredelung mit einer Veredelungsformulierung kann drucktechnisch erfolgen, insbesondere durch Bedrucken der Oberfläche mit Druckfarben. Darüber hinaus bezieht sich das erfindungsgemäße Verfahren aber auch auf andere Formen der Oberflächenveredelung, insbesondere das Aufbringen eines strahlungshärtbaren Klarlacks, wie er beispielsweise zum Schutz von bereits bedruckten Verpackungen gebräuchlich ist. Solche Klarlackschichten unterscheiden sich von farbigen Drucken im wesentlichen dadurch, daß sie im Gegensatz zu Druckfarben keine Pigmente enthalten. Nachfolgend wird beispielhaft auf das Drucken mittels strahlenhärtenden Druckfarben Bezug genommen. Hierdurch soll jedoch die allgemeine Anwendbarkeit der Erfindung auch für das Aufbringen von pigmentierten oder pigmentfreien Oberflächenveredelungen mit drucktechnischen oder anderen Applikationsverfahren, insbesondere in einem Schichtstärkenbereich zwischen 0,1 $\mu$m und 200 $\mu$m, nicht beschränkt werden.

Für zahlreiche Anwendungszwecke, beispielsweise im Verpackungsdruck, werden strahlenhärtende Druckfarbenformulierungen verwendet. Sie enthalten als wesentliche Bestandteile ihres Bindemittelsystems Monomere oder Oligomere, die unter Einwirkung von UV- oder Elektronenstrahlung polymerisieren und vernetzen. Dadurch wird eine besonders widerstandsfähige Druckoberfläche auch auf problematischen Oberflächen, beispielsweise auf Aluminiumschichten, erreicht. Gebräuchlich sind vor allem Veredelungsformulierungen auf Basis von Acrylatharzen und Epoxydharzen. Wegen ihrer positiven Eigenschaften haben strahlungshärtende Druckfarben seit etwa 20 Jahren große praktische Bedeutung erlangt. Weitere Anwendungsgebiete der vorliegenden Erfindung, zusätzlich zur Druckereitechnik, schließen die holzverarbeitende Industrie ein, beispielsweise beim Veredeln der Oberflächen von Parkettelementen.

Die Qualität einer strahlengehärteten Druckfarbenschicht wird entscheidend durch ihren Gehalt an nicht vernetzten migrationsfähigen Bestandteilen (Monomeren oder Oligomeren) beeinflußt. Der Gesamtgehalt dieser migrationsfähigen Komponenten in einer Schicht wird als "Globalmigration" bezeichnet.

Der Vernetzungsgrad und damit der Restanteil migrationsfähiger Substanzen ist entscheidend für die mechanische Widerstandsfähigkeit und allgemein für die technischen Eigenschaften der Schicht. Auch im Hinblick auf eventuelle Gesundheitsrisiken, die mit einem unzureichenden Vernetzungsgrad bei bestimmten Anwendungsfällen (Lebensmittelverpackungen) verbunden sein können, ist die zuverlässige Kontrolle der Schichtqualität von großer Bedeutung. Es sind deshalb schon zahlreiche unterschiedliche Verfahren für die Qualitätsprüfung vorgeschlagen worden.

Gebräuchlich ist insbesondere die HPLC-Analyse, bei der aus einer Probe des bedruckten Materials die unvernetzten Bestandteile, beispielsweise mit Methanol im Ultraschallbad, flüssig extrahiert und anschließend mit dem HPLC-Verfahren analysiert werden. Bei einem anderen gebräuchlichen Verfahren wird eine Probe des bedruckten Materials auf eine verhältnismäßig hohe Temperatur von beispielsweise 80 °C erhitzt und die dabei aus der Schicht frei werdenden Substanzen werden in der Gasphase gaschromatographisch (insbesondere mit Hilfe der GC-head space-Analyse) untersucht.

Die bisher bekannten Verfahren sind jedoch alle insoweit nachteilig, als sie destruktiv sind, d.h. die Entnahme eines Probstückes aus dem bedruckten Material erfordern. Das Probstück wird bei der Untersuchung zerstört. Vor allem aber ist keine Qualitätsprüfung ohne Unterbrechung der Produktion möglich. Außerdem erfordern die bekannten Analysemethoden aufwendige Apparaturen.

Der Erfindung liegt daher die Aufgabe zugrunde, die Qualitätskontrolle von strahlungsgehärteten Oberflächenveredelungen (insbesondere Druckfarbenschichten) nicht-destruktiv und mit verringertem Aufwand zu ermöglichen.

Die Aufgabe wird bei einem Verfahren der eingangs bezeichneten Art dadurch gelöst, daß die Veredelungsformulierung eine mit der Ionenbeweglichkeitsspektrometrie nachweisbare protonenaffine Indikatorsubstanz enthält und die aus der bedruckten Oberfläche nach der Strahlenhärtung entweichende Indikatorsubstanz mittels der Ionenbeweglichkeitsspektrometrie nachgewiesen wird.

Die Ionenbeweglichkeitsspektrometrie (ion mobility spectroscopy-IMS) ist eine Analysemethode, bei der die Moleküle der zu analysierenden Substanz chemisch bei Atmosphärendruck zu Molekülionen ionisiert und diese in einem Driftraum entsprechend ihren unterschiedlichen Beweglichkeiten mittels eines elektrischen Feldes räumlich getrennt werden.

Zur Analyse mit dem IMS-Verfahren werden Ionenbeweglichkeitsspektrometer verwendet. Sie bestehen im wesentlichen aus einer Ionenquelle, einem Ioneneinlaßgitter und einem Driftraum mit einer Fängerelektrode (Kollektor) sowie der erforderlichen Meßelektronik. Die zu analysierenden Moleküle werden mit einem Trägergas -in der Praxis meist Luft- in die Ionenquelle gesaugt. Dort werden die Trägergas-Moleküle durch Bestrahlung mit $\beta$-Teilchen einer Tritiumstrahlungsquelle oder $Ni^{63}$-Quelle ionisiert und dadurch sogenannte Reaktionsionen gebildet. Im Falle von Luft sind dies vor allem die Ionen $(H_2O)_6 H^+$ oder $(H_2O)_3 O_2{}^-$. In dem

2

an die Ionenquelle anschließenden Reaktionsraum reagieren die Reaktionsionen mit den nachzuweisenden Molekülen und bilden sogenannte Produktionen, d.h. ionisierte Moleküle.

Die Ionenquelle, der Reaktionsraum und der Driftraum werden üblicherweise von einer geraden Röhre gebildet, an der ein elektrisches Feld anliegt. Dieses Feld transportiert die Produktionen (und auch nicht reagierte Reaktionsionen) entlang der Röhre in Richtung auf den Kollektor. Am Eingang des eigentlichen Driftraumes befindet sich ein Ioneneinlaßgitter, welches beispielsweise alle 30 msec für ca. 300 $\mu$sec geöffnet wird. Die dabei jeweils in den Driftraum eindringende Ionenwolke wird bei ihrer Bewegung durch den Driftraum entsprechend der Beweglichkeit der Ionen im elektrischen Feld entmischt. Beim Auftreffen auf den Kollektor erzeugt jede Ionenart gleicher Beweglichkeit einen elektrischen Peak. Die Gesamtheit aller Peaks wird als Ionenbeweglichkeitsspektrum bezeichnet.

Die Beweglichkeit der Molekülionen wird in erster Linie durch deren Masse, also ihr Molekulargewicht, bestimmt. Daneben ist aber auch die Molekülstruktur zu berücksichtigen. Die Position der Peaks entlang der Drift-Zeitachse des Spektrums ist somit ein Maß für die Ionenbeweglichkeit der Produktionen. Die Peakfläche ist ein Maß für deren Anzahl, d.h. die Konzentration von Produktionen jeweils gleicher Beweglichkeit.

Nähere Einzelheiten zu dem IMS-Verfahren sind - sowohl hinsichtlich der apparativen, als auch hinsichtlich der chemisch-physikalischen Aspekte - der einschlägigen Literatur zu entnehmen. Ein Überblick wird beispielsweise gegeben in: G. Arnold, H.R. Döring "Ionenbeweglichkeitsspektrometrie, ZFI-Mitteilungen Nr. 154 (Zentralinstitut für Isotopen- und Strahlenforschung der Akademie der Wissenschaften der DDR) Leipzig (1990), 13-35 mit zahlreichen weiteren Literaturnachweisen.

Nachdem das IMS-Verfahren in den 70iger Jahren zunächst mit großem Interesse in wissenschaftlichen Fachkreisen aufgenommen wurde, hat sich in der Folgezeit gezeigt, daß die analytische Anwendungsbreite des Verfahrens recht eingeschränkt ist. Praktische Anwendung findet es insbesondere zum Aufspüren von phosphororganischen Industriegiften, Explosivstoffen und Drogen sowie im militärischen Bereich zum Aufspüren von Kampfstoffen. Für solche Anwendungszwecke sind tragbare Ionenbeweglichkeits-Spektrometer verfügbar, beispielsweise das Produkt RAID-1 der Bruker-Saxonia-Analytik-GmbH, Leipzig, Deutschland.

Eine Untersuchung über die Anwendbarkeit der Ionen-Mobilitäts-Spektrometrie zum Nachweis von Spuren migrierfähiger Komponenten in strahlengehärteten Druckfarben wurde veröffentlicht in Tagungsband 17. Münchener Klebstoff- und Veredelungsseminar 1992, Herausgeber Prof. Dr. Nitzl, Fachhochschule München. Dabei wurden verschiedene elektronenaffine Testsubstanzen, welche gebräuchliche Monomere von elektronenstrahlenhärtenden Druckfarbenzubereitungen sind, in Abhängigkeit von der Bestrahlungsdosis mit dem IMS-Verfahren analysiert. Die Ergebnisse zeigen einen Anstieg der Monomer-Konzentration mit zunehmender Strahlungsdosis. Dieses Analyseergebnis entspricht nicht den tatsächlichen Verhältnissen. Sowohl Vergleichsanalysen mit konventionellen Verfahren, als auch die praktische Erfahrung zeigen, daß die Vernetzung mit zunehmender Strahlungsdosis zunimmt und dementsprechend die Monomer-Konzentration abnimmt. Der Autor selbst äußert am Ende der Publikation Zweifel, ob das IMS-Verfahren zum Nachweis von Spuren migrierfähiger Komponenten in strahlengehärteten Druckfarben geeignet ist.

Gemäß der vorliegenden Erfindung enthalten Druckfarbenformulierungen, deren Qualität mit dem IMS-Verfahren überprüft werden soll, eine protonenaffine Indikatorsubstanz. Vorzugsweise ist ihre Protonenaffinität höher als die von Wasser (707 kJ/mol). Besonders bevorzugt soll die Protonenaffinität deutlich größer als die des Wassers sein, wobei Werte oberhalb von 750 kJ/mol, insbesondere oberhalb von 800 kJ/mol in besonderem Maße die Eignung als Indikatorsubstanz in Zubereitungen für strahlenhärtende Oberflächenveredelungen begründen. Es ist davon auszugehen, daß unter diesen Umständen ein Protonentransfer zwischen den Reaktionsionen R und den Molekülen M der Indikatorsubstanz stattfindet gemäß der Formel

$$RH^+ + M \,\text{-->}\, R + MH^+$$

Überraschenderweise hat sich gezeigt, daß die Konzentration der Indikatorsubstanz in der Veredelungsformulierung bestimmte Höchstwerte nicht überschreiten sollte. Sie sollte so bemessen sein, daß die Gleichgewichtskonzentration der Indikatorsubstanz in der Gasphase unmittelbar über der bedruckten und gehärteten Oberfläche höchstens 10 mg/m$^3$, bevorzugt höchstens 1 mg/m$^3$ beträgt. Dadurch wird der dynamische Bereich des IMS-Verfahrens im Rahmen der Erfindung optimal genutzt. In der Praxis hat sich gezeigt, daß die maximale Konzentration der Indikatorsubstanz in der Veredelungsformulierung höchstens 2 %, bevorzugt höchstens 1 %, besonders bevorzugt höchstens 0,5 % betragen sollte.

Insbesondere für radikalisch oder radikalisch/kationisch (hybrid) härtende Druckfarben haben sich Bestandteile des Initiatorsystems als besonders geeignete Indikatorsubstanzen erwiesen. Das Initiatorsystem dient in der Druckfarbenformulierung dazu, die vernetzende Polymerisation zu initiieren. Es kann aus nur einer Verbindung, nämlich einem Photoinitiator, oder aus mindestens zwei miteinander bei der Initiierung der Polymerisation reagierenden und zusammenwirkenden Substanzen bestehen. Da das Initiatorsystem, Monomere und (gegebenenfalls) Oligomere gemeinsam das Bindemittel der Druckfarbe bilden, werden sie insgesamt auch als Bindemittelsystem bezeichnet.

Ein Photoinitiator ist insbesondere bei der UV-Strahlenhärtung erforderlich. Er besteht aus Molekülen, die unter Einwirkung der UV-Strahlung in einen angeregten Zustand überführt werden, wobei im Falle radikalisch härtende Druckfarbenzubereitungen Photoinitiatoren verwendet werden, die unter Einwirkung von UV-Strahlen ein Radikalpaar bilden. Diese Radikale initiieren die Vernetzungs-Kettenreaktion der in der Druckfarbenzubereitung enthaltenen Monomere und Oligomere zu einer vernetzten und damit gehärteten Polymerisat-Oberflächenschicht.

Bei aus mehreren Substanzen bestehenden Initiator-Systemen bildet ein Molekül infolge der (UV- oder Elektronen)-Strahlung einen angeregten Zwischenzustand (beispielsweise einem relativ stabilen Triplett-Zustand), der selbst nicht zu einer Radikalbildung führt. Vielmehr ist eine weitere Substanz, nämlich ein sogenannter Coinitiator gegenwärtig, der mit dem angeregten Zustand des ersten Moleküls (Triplett-Zustand) reagiert und ein Radikalpaar bildet. Bei einem solchen System zur Vernetzungsinitiierung ist der Coinitiator in besonderem Maße als Indikatorsubstanz für die vorliegende Erfindung geeignet.

Die besondere Eignung von Photoinitiatoren und Coinitiatoren (allgemeinen Bestandteilen des Initiator-Systems) als Indikatorsubstanz für die zerstörungsfreie Qualitätsprüfung drucktechnisch aufgebrachter Veredelungen ist insofern besonders überraschend, als diese Substanzen nur in relativ geringen Konzentrationen von etwa 5 % in der Gesamtformulierung enthalten sind. Noch wesentlich kleiner ist der Rest-Gehalt dieser Substanzen in der fertigen Oberflächenveredelung, weil gerade die Bestandteile des Initiatorsystems bei der Polymerisation praktisch vollständig vernetzt werden. Die Konzentration in der fertigen Oberflächenveredelung liegt bei wenigen ppm. Dennoch haben sich gerade diese in extrem geringer Konzentration vorliegende Substanzen als geeignete Indikatoren für die Qualität der Oberflächenveredelung erwiesen.

Um die bei dem erfindungsgemäßen Verfahren bevorzugte geringe Konzentration der Indikatorsubstanz zu gewährleisten, ist es vielfach sogar bevorzugt, daß die Veredelungsformulierung zwei Initiatorsysteme enthält, nämlich ein erstes Initiatorsystem mit einem Photoinitiator oder Coinitiator hoher Protonenaffinität, der sich mit dem IMS-Verfahren nachweisen läßt und ein zweites Initiatorsystem, welches keine Komponenten mit ähnlich hoher Protonenaffinität enthält. Konkret sollte die Protonenaffinität sämtlicher Bestandteile des zweiten Initiatorsystems mindestens 10 % unter derjenigen der Indikatorsubstanz liegen. Durch die Verwendung von zwei Initiatorsystemen ist es möglich, daß einerseits die Indikatorsubstanz in der gewünschten geringen Konzentration von weniger als 2 %, vorzugsweise weniger als 1 % vorliegt, während andererseits der Gesamtgehalt an Initiatorsubstanzen in der Formulierung in der üblichen Höhe (meist bei ca. 5 %) liegt, um eine ausreichend vollständige Vernetzung zu gewährleisten.

Indikatorsubstanzen für die vorliegende Erfindung können recht unterschiedliche chemische Strukturen haben. Die Indikatorsubstanz für radikalisch oder radikalisch/kationisch härtende Druckfarben kann beispielsweise ein Benzoin, Benzoinether, Benzilketal, Thioxanthonderivat, $\alpha,-\alpha-$,Dialkoxyacetophenonderivat, $\alpha$-Hydroxyalkylphenon, $\alpha$-Aminoalkylphenonderivat, Acrylposphinoxid, Benzophenon oder ein Benzophenonderivat sein. In der Praxis besonders bewährt haben sich insbesondere die Substanzen 2-Hydroxy-2-methyl-1-phenyl-propan-1-on oder 1-(4-Isopropylphenyl)-2-hydroxy-2-methyl-propan-1-on. Weitere vorteilhafte Substanzgruppen sind Aminoacrylate, copolymerisierbare Amine und Alkylamine.

Für kationisch härtende Druckfarben sind nach den bisher vorliegenden Erkenntnissen Alkencarbonate, insbesondere Propylencarbonat, sowie Arylsulfoniumsalze, Aryljodoniumsalze und Vinylether geeignet.

Das Molgewicht der Indikatorsubstanz sollte vorzugsweise zwischen 50 und 400 liegen.

Die Erfindung wird im folgenden anhand von in den Figuren schematisch dargestellten Ausführungsbeispielen näher erläutert, es zeigen:

Fig. 1     eine perspektivische Prinzipdarstellung einer Meßanordnung gemäß der Erfindung,

Fig. 2     eine Schnittdarstellung eines für die Erfindung geeigneten Ionenbeweglichkeitsspektrometers,

Fig. 3     eine graphische Darstellung der zeitlichen Änderung eines an einer strahlengehärteten Druckfarbenschicht gemessenen IMS-Spektrums,

Fig. 4     eine Gegenüberstellung von Meßergebnissen, die einerseits mit konventionellen Methoden und andererseits mit der Erfindung gewonnen wurden.

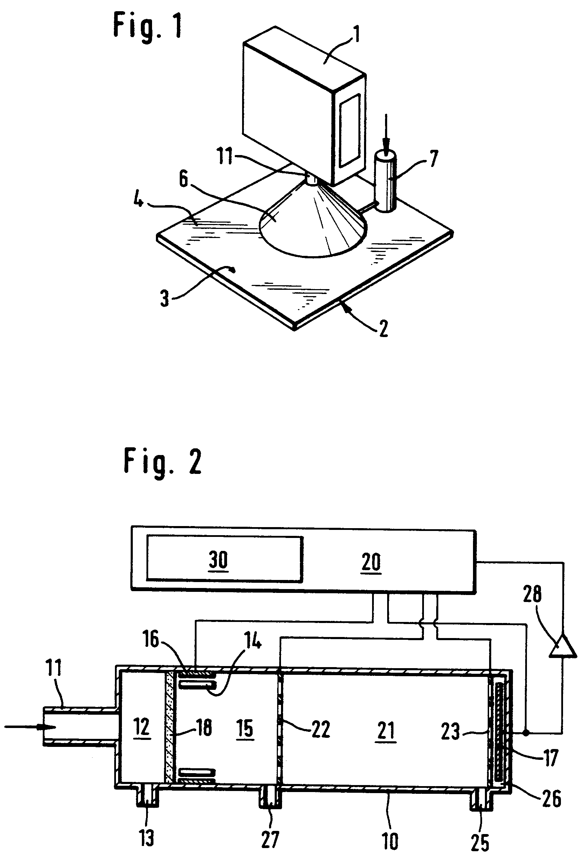

In Fig. 1 ist ein Ionenbeweglichkeitsspektrometer 1 dargestellt, welches sich in der Meßposition über einem Druckerzeugnis 2 befindet, auf dessen Oberfläche 3 eine strahlungsgehärtete Oberflächenveredelung (insbesondere Druckschicht) 4 aufgebracht ist, deren Qualität geprüft werden soll. Dem Analysegas-Einlaß 11 des Ionenbeweglichkeitsspektrometers 1 ist ein Ansaugkopf 6 vorgeschaltet.

Der Ansaugkopf 6 hat im dargestellten Fall die Form eines stumpfen Kegels, dessen unterer, die größere Kegelöffnung (Ansaugöffnung) begrenzender Rand auf der Oberfläche 3 des Druckerzeugnisses 2 aufliegt. Um die plane Auflage der Ansaugöffnung des Ansaugkopfes 6 auf der Oberfläche 3 zu gewährleisten, liegt das Druckerzeugnis an der Meßstelle auf einer nicht dargestellten ebenen Auflage auf. Die obere kleinere Öffnung des Ansaugkopfes 6 ist gasdicht an den Analysegas-Einlaß 11 des Spektrometers 1 angeschlossen. Selbstverständlich kann der Ansaugkopf 6 auch anders gestaltet sein, solange einerseits der gasdichte Anschluß an das Spektrometer gewährleistet ist und andererseits sein Innenvolumen über eine hinreichend große Austauschfläche in Verbindung zu der Druckschicht steht, deren Qualität überprüft werden soll.

Das Innere des Ansaugkopfes 6 steht über einen Seitenanschluß in Verbindung zu einem Aktivkohle-Filter 7, durch den Luft als Trägergas angesaugt werden kann. Der Aktivkohle-Filter 7 dient dabei zur Reinigung der Luft.

In Fig. 2 erkennt man ein röhrenförmiges Spektrometergehäuse 10, in dessen Gaseinlaß 11 das Trägergas mit den darin transportierten Analyt-Molekülen in eine Membran-Vorkammer angesaugt wird. Die (nicht dargestellte) Gaspumpe zum Ansaugen des Gases ist über eine seitliche Anschlußöffnung 13 mit der Membran-Vorkammer 12 verbunden. Durch die seitliche Anschlußöffnung 13 kann nach Umschalten eines ebenfalls nicht dargestellten Magnetventils wahlweise auch Spülgas zugeführt werden, um die Membran-Vorkammer, den Gaseinlaß und den in Fig. 2 nicht dargestellten Ansaugkopf 6 zu spülen. Hierzu wird zweckmäßigerweise über einen weiteren nicht dargestellten Filter geführte, getrocknete und gereinigte Luft verwendet.

Die Vorkammer 12 wird ausgangsseitig von einer nichtporösen (homogenen) Membran 18 begrenzt. Sie besteht bevorzugt aus Silikongummi und dient vor allem dazu, das Eindringen von Wassermolekülen zu verhindern, während die Indikatormoleküle hindurchdiffundieren können. An die Membran 18 schließt sich der Reaktionsraum 15 an, an dessen Wänden eine $Ni^{63}$-Quelle 14 angeordnet ist, durch deren $\beta$-Strahlung aus dem Trägergas positiv geladene Reaktionsionen, insbesondere vom Typ $(H_2O)_nH^+$ gebildet werden. Zwischen einer ebenfalls in dem Reaktionsraum 15 vorgesehenen positiven Elektrode 16 und einer Kollektor-Elektrode 17 liegt eine Gleichspannung an, die von einer Hochspannungsquelle in der elektronischen Zentraleinheit 20 des Gerätes erzeugt wird. Dadurch besteht ein elektrisches Gleichspannungsfeld zwischen den Elektroden 16,17, das vorzugsweise durch (nicht dargestellte) dazwischenliegende Elektroden auf Zwischenpotentialen stabilisiert wird.

In der Reaktionskammer 15 reagieren die Reaktionsionen unter Ladungsübertragung mit den Analyt-Molekülen und bilden positiv geladene Produktionen. Die Produktionen und noch vorhandene Reaktionsionen werden von dem elektrischen Feld in Richtung auf den Kollektor 17 beschleunigt. Sie gelangen in den Driftraum 21, der von dem Reaktionsraum 15 durch ein Schaltgitter 22 getrennt ist. Am Ende des Driftraumes 21 befindet sich vor dem Kollektor 17 ein Schirmgitter 23, welches dazu dient Gegenladungen, die von der Ionenwolke erzeugt werden, möglichst definiert klein zu halten. In dem Driftraum 21 strömt den Ionen ein Driftgas entgegen, welches durch einen Driftgaseinlaß 25 am kollektorseitigen Ende 26 des Driftraumes 21 zugeführt und an einem in dem Reaktionsraum 15 in der Nähe des Schaltgitters 22 angeordneten Driftgasauslaß 27 abgeführt wird. Auch das Driftgas ist getrocknet und gereinigt. Es wird zweckmäßigerweise im Kreis mit einer nicht dargestellten Pumpe in einer kontrollierten Volumenströmung zirkuliert.

Zur Durchführung einer Analyse wird nach ausreichendem Spülen der Membran-Vorkammer 12 mit Spülgas Probengas in die Membran-Vorkammer 12 angesaugt. In der Probe enthaltene Moleküle der Indikatorsubstanz gelangen durch die Membran 18 in den Reaktionsraum 15 und werden dort ionisiert. Das Schaltgitter 22 wird in relativ langen Zeitabständen (beispielsweise 30 msec) jeweils für relativ kurze Perioden (beispielsweise 300 $\mu$sec) geöffnet. Bei jeder Öffnung des Gitters 22 gelangt eine Ionenwolke in den Driftraum 21. Die darin enthaltenen Moleküle entmischen sich während der Driftbewegung durch den Driftraum 21 entsprechend ihren Beweglichkeiten, wobei die am schnellsten beweglichen (kleinsten) Moleküle zuerst und die am wenigstens beweglichen (großen) Moleküle zuletzt an dem Kollektor 17 ankommen. An dem Kollektor 17 werden die Ionen entladen, so daß ein elektrischer Strom fließt, der über einen Verstärker 28 verstärkt und der elektronischen Zentraleinheit 20 zugeführt wird. Diese enthält geeignete elektronische Weiterverarbeitungs-Elemente, insbesondere in Form eines Mikrocomputers, der die Signale - in der Regel nach Digitalisierung softwaremäßig gesteuert - weiterverarbeitet und die Ergebnisse auf einem Display 30 in graphischer Form oder als Zahlenwerte anzeigt. Selbstverständlich ist auch eine Ausgabe über einen Drucker möglich.

In der Praxis wird zur Aufnahme eines Spektrums das Schaltgitter viele Male (zum Beispiel sechzehnmal) geöffnet und wieder geschlossen. Ein Ionenbeweglichkeitsspektrum wird durch Akkumulation der dabei gewonnen Daten gebildet.

Eine weiter detaillierte Erläuterung des IMS-Meßverfahrens ist nicht erforderlich, weil die bei der Erfindung angewandte Meßtechnik weitgehend konventionell ausgebildet ist. Eine Besonderheit besteht neben der bereits erwähnten Ansaugkopf 6 darin, daß die Membran 18, die die Membranvorkammer 12 von

5

dem Reaktionsraum 15 trennt, auf eine relativ hohe Temperatur von mindestens etwa 60°C, bevorzugt mindestens etwa 70°C, aufgeheizt wird. Die Membran dient dazu die Selektivität des Spektrometers zu verbessern und störende Substanzen abzuhalten. Durch die Erhöhung der Temperatur kann die Meßgeschwindigkeit angehoben werden. Dies hat sich bei der Analyse von Druckfarben im Hinblick auf die hier gegebenen Empfindlichkeits- und Geschwindigkeitsanforderungen als besonders vorteilhaft erwiesen.

Zur Erhöhung der Meßgeschwindigkeit kann es vorteilhaft sein, das Trägergas, welches zur Aufnahme der Indikatormoleküle über die Oberfläche 3 des Druckerzeugnisses 2 geführt wird, vorzuheizen. Besonders energie-ökonomisch kann dies geschehen, in dem das beim Spülen an der heißen Membran 18 vorbeigeführte und deswegen erhitzte Gas auf einem nicht dargestellten Rückführungsweg in den Ansaugkopf 6 über die Oberfläche 3 gepumpt wird.

Zur Durchführung einer Prüfung wird, wie bereits erläutert, der Ansaugkopf 6 mit seiner Ansaugöffnung auf das Druckerzeugnis aufgelegt (Figur 1). Wenn die Prüfung im statischen Zustand erfolgt, läßt sich eine ausreichende Abdichtung dadurch erreichen, daß das Druckerzeugnis 2 auf einer ebenen Unterlage liegt.

Besonders bevorzugt erfolgt die Prüfung im On-Line-Betrieb, d.h. im laufenden Druckprozeß über der bewegten Druckbahn. Dabei kann bei geringen Bahngeschwindigkeiten der Ansaugkopf 6 auf die bewegte Druckbahn 2 aufgesetzt und während der Messung mitbewegt werden.

Bei höheren Druckgeschwindigkeiten ist dies jedoch nicht praktikabel. In diesem Fall wird der Ansaugkopf 6 mit seiner Ansaugöffnung knapp über der bewegten Druckbahn 2 stationär angeordnet. Gemäß einer bevorzugten Ausführungsform der Erfindung sind dabei am Rand der Ansaugöffnung Mittel vorgesehen, durch die das Eindringen von Gas aus dem den Ansaugkopf umgebenden Außenraum in den Innenraum des Ansaugkopfes (also durch den Spalt zwischen dem Rand des Ansaugkopfes 6 und dem Druckerzeugnis 2) reduziert wird.

Diese Mittel können in unterschiedlicher Weise realisiert sein. Insbesondere kann der Rand der probenseitigen Ansaugöffnung mit einem Streifen aus einem elastisch nachgiebigen und reibungsarmen Material, wie beispielsweise Teflon, umgeben sein, der so ausgebildet ist, daß er bei der Messung einen den Spalt weitestgehend verschließenden, auf der Oberfläche des Druckerzeugnisses 2 schleifenden Abdichtungsvorhang bildet.

Alternativ kann ein Gasvorhang verwendet werden, um das Eindringen störender Gase aus dem Außenraum in den Innenraum des Ansaugkopfes 6 zu verhindern. Zu diesem Zweck sind gemäß einer weiteren bevorzugten Ausführungsform an dem Rand der Ansaugöffnung Gasdüsen derartig angeordnet, daß ein den Rand vollständig umgebender Gasvorhang erzeugt werden kann. In der praktischen Realisierung kann beispielsweise der Ansaugkopf mit einem an dem Rand seiner Ansaugöffnung umlaufenden Rohr versehen sein, welches auf der probenseitigen Seite Bohrungen als Gasdüsen aufweist. Zur Erzeugung des Gasvorhanges kann beispielsweise gereinigte und getrocknete Luft oder ein Inertgas verwendet werden.

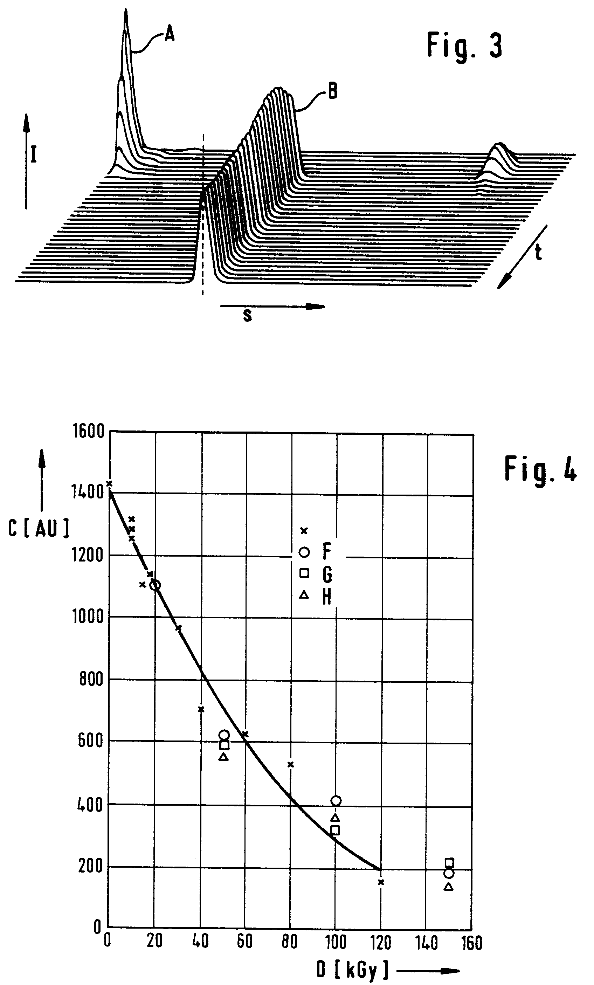

Fig. 3 zeigt in einer dreidimensionalen graphischen Darstellung Ionenbeweglichkeitsspektren, die an einem elektronenstrahlengehärteten Rollenoffset-Versuchsdruck gemessen wurden. Dabei war der Druckfarbenformulierung als Indikator 0,5 % des Produktes AGeflexFM2 der Firma CPS, Cray Valley, USA, zugesetzt. Die zur Härtung eingesetzte Strahlungsdosis betrug 18 kGy.

In der Figur sind IMS-Spektren (Kollektorstrom I gegen Laufzeit s) aufgetragen, die über einen Zeitraum von insgesamt 2 Minuten gemessen wurden.

Die ursprünglich in einem geschlossenen Probengefäß vorhandenen Reaktionsionen sind leicht und beweglich und erzeugen deswegen einen großen Peak A bei kurzen Laufzeiten. Nach dem Einbringen einer 50 cm$^2$ großen Druckprobe in das an das Ionenbeweglichkeitsspektrometer angeschlossene Probengefäß reagieren sie innerhalb von 20 sec mit dem Indikator. Infolgedessen verschwindet der Reaktionsionenpeak A und es entsteht ein Produktionenpeak B, dessen Fläche der Konzentration des Indikators in der Umgebungsluft der Druckprobe entspricht. Infolge der schnellen Bildung der Produktionen und ihrer unmittelbaren Meßbarkeit kann die Indikatorkonzentration innerhalb von weniger als 10 sec gemessen werden. Dies entspricht üblichen Einstellzeit-Zeitkonstanten von Druckmaschinen, mit denen strahlenhärtende Druckfarben verarbeitet werden.

Damit zeigt sich das Potential der Erfindung zur real time-Qualitätskontrolle während der Produktion.

In Fig. 4 ist die Dosisabhängigkeit des IMS-Indikatorpeaks B dargestellt, wobei die Abszisse die Strahlendosis in kGy zeigt, während auf der Ordinate die Peakfläche, d.h. die Konzentration des Indikators in willkürlichen Einheiten dargestellt ist. Experimentell bestimmte Werte der Indikator-Peakfläche sind als Kreuze eingetragen.

Ebenfalls in Fig. 4 eingetragen, sind HPLC-Analysewerte für drei unterschiedliche typische Modell-Bindemittelsysteme F,G,H, deren Zusammensetzung in der nachfolgenden Tabelle 1 angegeben ist.

6

EP 0 683 392 A2

Tabelle 1

| Zusammensetzung in Gewichtsprozent | | | | | |
|---|---|---|---|---|---|
| Komponente | Markenname | Hersteller | F | G | H |
| TPGDA | TPGDA | UCB | 13.0 | 9.8 | 11.4 |
| TMPTA | TMPTA | UCB | 26.1 | 9.8 | 11.4 |
| HEMA | HEMA | UCB | 4.3 | 1.7 | 1.9 |
| Acrylic acid dimer | $\beta$-CEA | UCB | -- | 25.0 | -- |
| Novolac acrylate | RD202 | UCB | 56.6 | 21.2 | 27.0 |
| Aminoacrylate | Ageflex FA-1 | CPS | -- | -- | 10.0 |
| Acrylated acrylic | EB1701 | UCB | -- | 32.5 | 38.3 |

Die Zusammensetzung F entspricht dem Bindemittelsystem der Druckfarbe, an dem die in Fig. 3 dargestellten Messungen vorgenommen wurden.

Sämtliche Konzentrationswerte, also sowohl die mit der HPLC ermittelten Konzentrationen für die Modellsubstanzen F,G und H, als auch die Peakflächen des IMS-Indikators wurden bei einer Dosis von 20 kGy auf den gleichen Wert der willkürlichen Skalenteilung (1100) normiert.

Fig. 4 zeigt, daß nicht nur die drei Modellsubstanzen F,G und H untereinander die gleiche charakteristische Abhängigkeit von der zur Härtung verwendeten Strahlungsdosis zeigen, sondern daß die Meßergebnisse des erfindungsgemäßen Verfahrens mit vergleichbarer Meßgenauigkeit den gleichen funktionalen Verlauf zeigen. Damit ist dokumentiert, daß auf Basis der Erfindung ein Qualitätsparameter für die Vernetzung von Druckfarbenschichten gemessen wird, der hervorragend mit konventionell bestimmten HPLC-Werten korreliert, jedoch einen wesentlich geringeren apparativen Aufwand erfordert und zerstörungsfrei unmittelbar im Produktionsprozeß gemessen werden kann.

**Patentansprüche**

1. Verfahren zum qualitätskontrollierten Veredeln einer Oberfläche mit einer strahlungsgehärteten Oberflächenveredelung, bei welchem eine Veredelungsformulierung auf die Oberfläche aufgetragen und unter Einwirkung von Strahlung polymer vernetzt wird, **dadurch gekennzeichnet,** daß die Veredelungsformulierung eine mit der Ionenbeweglichkeitsspektrometrie nachweisbare protonenaffine Indikatorsubstanz enthält und die aus der bedruckten Oberfläche nach der Strahlenhärtung entweichende Indikatorsubstanz mittels der Ionenbeweglichkeitsspektrometrie nachgewiesen wird.

2. Verfahren nach Anspruch 1**, dadurch gekennzeichnet,** daß die Protonenaffinität der Indikatorsubstanz größer als die Protonenaffinität von Wasser ist.

3. Verfahren nach Anspruch 1 oder 2, **dadurch gekennzeichnet,** daß die Protonenaffinität der Indikatorsubstanz mindestens 750 kJ/mol, bevorzugt mindestens 800 kJ/mol beträgt.

4. Verfahren nach einem der vorhergehenden Ansprüche, **dadurch gekennzeichnet,** daß die Konzentration der Indikatorsubstanz in der Veredelungsformulierung so bemessen ist, daß ihre Gleichgewichtskonzentration in der Gasphase über der bedruckten Oberfläche nach der Strahlenhärtung höchstens 10 mg/m$^3$, bevorzugt höchstens 1 mg/m$^3$ beträgt.

5. Verfahren nach einem der vorhergehenden Ansprüche, **dadurch gekennzeichnet,** daß die Konzentration der Indikatorsubstanz in der Veredelungsformulierung höchstens 2 %, bevorzugt höchstens 1 %, besonders bevorzugt höchstens 0,5 % beträgt.

6. Verfahren nach einem der vorhergehenden Ansprüche, **dadurch gekennzeichnet,** daß die Indikatorsubstanz ein Bestandteil des Initiatorsystems der Veredelungsformulierung, insbesondere ein Photoinitiator oder ein Coinitiator ist.

7. Verfahren nach Anspruch 6, **dadurch gekennzeichnet,** daß die Veredelungsformulierung ein weiteres Initiatorsystem enthält, wobei alle Bestandteile des zweiten Initiatorsystems eine um mindestens 10 % geringere Protonenaffinität als die Indikatorsubstanz haben.

7

8. Verfahren nach einem der vorhergehenden Ansprüche, **dadurch gekennzeichnet,** daß das Molekulargewicht der Indikatorsubstanz mindestens 50 und höchstens 400 beträgt.

9. Verfahren nach einem der vorhergehenden Ansprüche, **dadurch gekennzeichnet,** daß die Indikatorsubstanz ein Benzoin, Benzoinether, Benzilketal, Thioxanthonderivat, $\alpha,-\alpha-$,Dialkoxyacetophenonderivat, $\alpha$-Hydroxyalkylphenon, $\alpha$-Aminoalkylphenonderivat, Acylposphinoxid, Benzophenon, ein Benzophenonderivat, ein Aminoacrylat, ein copolymerisierbares Amin oder ein Alkylamin, insbesondere 2-Hydroxy-2-methyl-1-phenyl-propan-1-on oder 1-(4-Isopropylphenyl)-2-hydroxy-2-methyl-propan-1-on ist.

10. Strahlungshärtende Veredelungsformulierung zum qualitätskontrollierten Veredeln einer Oberfläche gemäß einem Verfahren nach einem der vorhergehenden Ansprüche, **dadurch gekennzeichnet,** daß sie eine protonenaffine, mittels Ionenbeweglichkeitsspektrometrie nachweisbare Indikatorsubstanz enthält.

11. Vorrichtung zur Qualitätskontrolle von Oberflächenveredelungen gemäß einem Verfahren nach einem der Ansprüche 1 bis 8**, dadurch gekennzeichnet,** daß sie ein Ionenbeweglichkeitsspektrometer (1) mit einem Gaseinlaß (11) einschließt und dem Gaseinlaß (11) ein Ansaugkopf (6) vorgeschaltet ist, der über eine erste kleinere Öffnung an den Gaseinlaß 11 angeschlossen ist und als zweite größere Öffnung eine Ansaugöffnung aufweist, deren Rand so ausgebildet ist, daß er mit minimalem Abstand zu der Oberfläche des Druckerzeugnisses (2) positionierbar ist.

12. Vorrichtung nach Anspruch 11, **dadurch gekennzeichnet,** daß der Reaktionsraum (15) des Ionenbeweglichkeitsspektrometers (1) in Richtung auf den Gaseinlaß (11) von einer Membran (18) abgeschlossen ist, die im Betrieb des Ionenbeweglichkeitsspektrometers auf eine Temperatur von mindestens 60°C, bevorzugt mindestens 70°C beheizt wird.

13. Vorrichtung nach einem der Ansprüche 11 oder 12, **dadurch gekennzeichnet,** daß an dem Rand der größeren Öffnung des Ansaugkopfes Mittel vorgesehen sind, durch die das Eindringen von Gas aus dem den Ansaugkopf umgebenden Außenraum in den Innenraum des Ansaugkopfes reduziert wird.

14. Vorrichtung nach Anspruch 13, **dadurch gekennzeichnet,** daß die Mittel zum Reduzieren des Eindringens von Gas einen an dem Rand der größeren Öffnung des Ansaugkopfes umlaufenden Streifen aus einem nachgiebigen und reibungsarmen Material einschließen.

15. Vorrichtung nach Anspruch 13 oder 14, **dadurch gekennzeichnet,** daß die Mittel zum Reduzieren des Eindringens von Gas Gasdüsen zur Erzeugung eines Gasvorhanges einschließen, die an dem Rand der Ansaugöffnung des Ansaugkopfes 6 umlaufend angeordnet sind.

Fig. 1

Fig. 2

# Fig. 3

# Fig. 4